(19) Europäisches Patentamt
European Patent Office
Office européen des brevets

(11) Veröffentlichungsnummer: **0 163 260**
**B1**

# (12) EUROPÄISCHE PATENTSCHRIFT

(45) Veröffentlichungstag der Patentschrift: **03.08.88**

(21) Anmeldenummer: **85106343.8**

(22) Anmeldetag: **23.05.85**

(51) Int. Cl.[4]: **C 07 D 207/267**, C 07 D 207/27, C 07 D 401/12, C 07 D 403/12, C 07 D 413/12, A 61 K 31/40, A 61 K 31/395, A 61 K 31/495, A 61 K 31/535

## (54) Neue substituierte Pyrrolidinone, Verfahren zu ihrer Herstellung und Arzneimittel.

(30) Priorität: **30.05.84 DE 3420193**

(43) Veröffentlichungstag der Anmeldung: **04.12.85 Patentblatt 85/49**

(45) Bekanntmachung des Hinweises auf die Patenterteilung: **03.08.88 Patentblatt 88/31**

(84) Benannte Vertragsstaaten: **AT BE CH DE FR GB IT LI LU NL SE**

(56) Entgegenhaltungen:
**CH - A - 629 771**

**JOURNAL OF THE AMERICAN CHEMICAL SOCIETY, Band 72,März 1950 P.L. PAYTASH et al. "The reaction of Itaconic Acid with Primary Amines" Seiten 1415, 1416**
**Drug Development Res. 2, 464 (1982)**

(73) Patentinhaber: **BOEHRINGER INGELHEIM KG, D-6507 Ingelheim am Rhein (DE)**
(84) Benannte Vertragsstaaten: **BE CH DE FR IT LI LU NL SE AT**

(73) Patentinhaber: **Boehringer Ingelheim International G.m.b.H, D-6507 Ingelheim am Rhein (DE)**
(84) Benannte Vertragsstaaten: **GB**

(72) Erfinder: **Weber, Karl-Heinz, Dr., Kaiser-Karl-Strasse 11, D-6535 Gau-Algesheim (DE)**
Erfinder: **Schneider, Claus, Dr., Albrecht-Dürer-Strasse 19, D-6507 Ingelheim/Rhein (DE)**
Erfinder: **Walther, Gerhard, Dr., Pfarrer-Heberer-Strasse 37, D-653 Bingen/Rhein (DE)**
Erfinder: **Hinzen, Dieter, Prof. Dr., Stromberger Strasse 36, D-653 Bingen/Rhein (DE)**
Erfinder: **Kuhn, Franz Josef, Dr., Beethovenstrasse 11, D-6535 Gau-Algesheim (DE)**
Erfinder: **Lehr, Erich, Dr., In der Toffel 5, D-6531 Waldalgesheim (DE)**
Erfinder: **Ensinger, Helmut, Dr., Im Herrengarten 10, D-6501 Wackernheim (DE)**
Erfinder: **Tröger, Wolfgang, Dr., St. Jakobus-Strasse 25, D-6534 Stromberg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft substituierte Pyrrolidinone, Verfahren zu ihrer Herstellung und Arzneimittel, die diese Verbindungen als Wirkstoff enthalten.

Die erfindungsgemässen Verbindungen dienen der Verbesserung der cerebralen Insuffizienz und haben sich in entsprechenden Tierversuchen als hoch wirksam erwiesen.

Aus der schweizerischen Patentschrift Nr. 629 771 sind N-(1′-Ethyl-2′-oxo-5-pyrrolidinylmethyl)-benzamide bekannt. Von den erfindungsgemäss beanspruchten Verbindungen unterscheiden sich diese durch Position und Art der Substituenten wie auch durch die angegebene pharmakologische Wirkungsrichtung.

Als strukturell ähnlich gebaute Nootropica sind das 1-Carbamoylmethyl-pyrrolidin-2-on (Pirazetam), das 1-(p-Methoxybenzoyl)-pyrrolidin-2-on (Anirazetam) und das 1-Carbamoylmethyl-4-hydroxy-pyrrolidin-2-on (Oxirazetam) bereits in der Literatur beschrieben, siehe B.J.R. Nicolaus, Drug Development Res. 2, 464 (1982), P.L. Paytasch, J. Amer. Chem. Soc. 72, 1415 (1950).

Es wurde nun gefunden, dass die Einführung einer Seitenkette mit einer Aminocarbonylfunktion in das Pyrrolidinonmolekül eine gegenüber den bekannten Stoffen wesentliche Verbesserung der Wirkung und darüber hinaus ein von den bekannten Verbindungen abgehobenes Wirkungsprofil aufweisen.

Gegenstand der Erfindung sind substituierte Pyrrolidinone der allgemeinen Formel

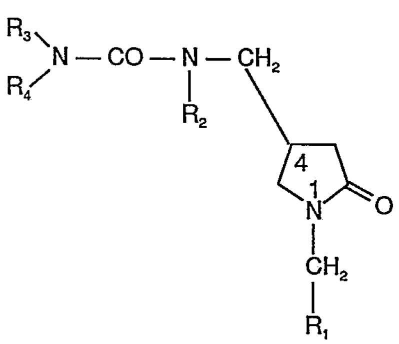

(I)

worin

$R_1$ einen Phenylrest, der ein- oder zweifach durch Methyl, Methoxy, Fluor, Chlor, Brom oder Trifluormethyl substituiert sein kann oder einen Pyridylrest;

$R_2$ Wasserstoff oder einen geradkettigen oder verzweigten Alkylrest mit 1-4 Kohlenstoffatomen;

$R_3$ einen geradkettigen oder verzweigten Alkylrest mit 1-3 Kohlenstoffatomen, einen Hydroxyalkylrest mit 2-3 Kohlenstoffatomen, einen Phenylrest, der ein- oder zweifach durch Chlor, Brom, Methyl oder Methoxy substituiert sein kann, einen Cyclohexylrest oder einen Dialkylaminoalkylrest, wobei jede Alkylgruppe 1-3 Kohlenstoffatome aufweisen kann;

$R_4$ Wasserstoff oder einen geradkettigen oder verzweigten Alkylrest mit 1-3 Kohlenstoffatomen oder

$R_3$ und $R_4$ zusammen mit dem Stickstoffatom einen Piperidin-, Morpholin- oder Piperazinring, wobei die Ringe durch ein bis zwei Methylgruppen substituiert sein können und der Piperazinring am Stickstoffatom in 4-Stellung auch eine Phenyl-, Chlorphenyl- oder Benzylgruppe tragen kann, oder den Nortropanylrest bedeuten.

Solche Endprodukte, die eine basische Funktion im Molekül besitzen, können mit Säuren physiologisch verträgliche Säureadditionssalze bilden. Als Säuren eignen sich hierfür sowohl anorganische Säuren wie Halogenwasserstoffsäuren, Schwefelsäure, Phosphorsäure und Aminosulfonsäure als auch organische Säuren wie Ameisensäure, Essigsäure, Propionsäure, Milchsäure, Glykolsäure, Glukonsäure, Maleinsäure, Fumarsäure, Bernsteinsäure, Weinsäure, Benzoesäure, Salicylsäure, Zitronensäure, Ascorbinsäure, p-Toluolsulfonsäure oder Oxyäthansulfonsäure. Die Überführung in die Säureadditionssalze erfolgt nach üblichen Methoden.

Bevorzugte Verbindungen der allgemeinen Formel I sind solche, bei denen $R_1$ den Phenylrest, $R_2$ Wasserstoff, $R_3$ einen Phenylrest, der gegebenenfalls in o- und/oder p-Stellung durch Chlor substituiert ist, $R_4$ Wasserstoff oder $R_3$ und $R_4$ zusammen mit dem Stickstoffatom einen gegebenenfalls ein- oder zweifach methylsubstituierten Morpholinyl-, Piperazinyl- oder Piperidinylrest bedeutet. Insbesondere von Interesse sind solche Verbindungen der allgemeinen Formel I, in denen $R_1$ Phenyl, $R_2$ Wasserstoff, $R_3$ Dialkylaminoalkyl mit jeweils 1-3 Kohlenstoffatomen oder p-Chlorphenyl und $R_4$ Wasserstoff oder $R_3$ und $R_4$ zusammen mit dem Stickstoffatom einen Piperazinring bedeuten, der gegebenenfalls in 4-Stellung durch Methyl substituiert ist. Insbesondere seien genannt die Verbindungen 4-(N-Methylpiperazinyl-carbonylaminomethyl)-1-benzylpyrrolidin-2-on und 4-(p-Chlorphenylamino-carbonylaminomethyl)-1-benzylpyrrolidin-2-on.

Gegenstand der Erfindung sind ferner die folgenden Verfahren zur Herstellung von Verbindungen der allgemeinen Formel I:

a) Umsetzung eines Aminomethylpyrrolidin-2-ons der allgemeinen Formel

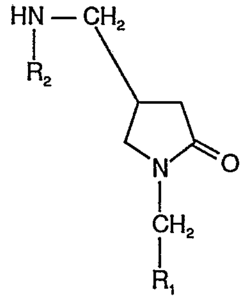

(II)

worin

$R_1$ und $R_2$ die oben angegebene Bedeutung besitzen, mit einem Isocyanat der allgemeinen Formel

$O{=}C{=}N{-}R_3$ (III)

worin

$R_3$ die oben angeführte Bedeutung mit Ausnahme der Bedeutung Hydroxyalkyl besitzt; nach diesem Verfahren erhält man solche Verbindungen der allgemeinen Formel I, in denen $R_4$ für Wasserstoff steht.

b) Umsetzung einer Verbindung der allgemeinen Formel II mit einem Chlorcarbonylamid der allgemeinen Formel

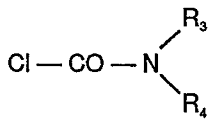 (IV)

worin

$R_3$ die in Anspruch 1 angegebene Bedeutung mit Ausnahme von Hydroxyalkyl und $R_4$ die in Anspruch 1 angegebene Bedeutung mit Ausnahme von Wasserstoff besitzen, umsetzt;

c) Umsetzung einer Verbindung der allgemeinen Formel II mit einem Chlorkohlensäureester der allgemeinen Formel

Cl-CO-O-Y (V)

worin

Y einen Alkylrest mit 1-4 Kohlenstoffatomen, den Benzyl-, Phenyl- oder p-Nitrophenylrest bedeutet, zu einem Carbamat der allgemeinen Formel

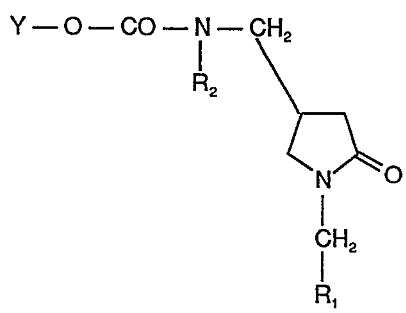 (VI)

worin

$R_1$, $R_2$ und Y die oben angeführte Bedeutung haben. Das Carbamat VI reagiert mit einem Amin der allgemeinen Formel

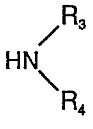 (VII)

worin

$R_3$ und $R_4$ die oben angegebene Bedeutung haben, zum gewünschten Endprodukt.

d) Ein Endprodukt der allgemeinen Formel I, worin $R_2$ und/oder $R_4$ Wasserstoff bedeuten, kann in üblicher Weise alkyliert werden.

Die Verfahren a) und b) werden in inerten wasserfreien Lösungsmitteln wie Dioxan, Tetrahydrofuran, Dimethylformamid, Äther, Benzol, Toluol, chlorierten Kohlenwasserstoffen usw. bei Temperaturen zwischen 0°C und der Siedetemperatur des Reaktionsgemisches durchgeführt; bevorzugt wird bei Raumtemperatur gearbeitet. Die Umsetzung einer Aminoverbindung der Formel II mit einem Isocyanat der Formel III kann auch ohne Lösungsmittel erfolgen.

Beim Verfahren b) empfiehlt sich der Zusatz eines säurebindenden Mittels, z.B. einer organischen Base wie Triäthylamin oder Pyridin oder eines Alkalicarbonats.

Das zweistufige Verfahren c) wird in der ersten Stufe, der Umsetzung eines Amins der Formel II mit einem Chlorkohlensäureester der Formel V, ebenfalls in einem wasserfreien inerten Lösungsmittel, wie oben angegeben, und bei Temperaturen zwischen 0°C und der Siedetemperatur des Reaktionsgemisches durchgeführt.

Die Reaktion zwischen einem intermediär gebildeten Carbamat der Formel VI und einem Amin der Formel VII erfolgt in wasserfreien inerten Lösungsmitteln, wie oben beispielsweise genannt, oder mit einem Überschuss an Amin der Formel VII, bei Temperaturen zwischen 0°C und Raumtemperatur oder mässig erhöhter Temperatur; bei niedrig siedenden Aminen ist die Verwendung eines Autoklaven angezeigt. Gewünschtenfalls kann der Reaktion eine tertiäre organische Base zum Abfangen des sich bildenden Phenols zugesetzt werden.

Die unter d) erwähnte Alkylierung erfolgt in üblicher Weise durch Salzbildung am Harnstoff mittels Natriumhydrid oder Natriummethylat und anschliessender Umsetzung mit einem Alkylhalogenid oder einem Dialkylsulfat.

Die neuen Verbindungen der allgemeinen Formel I besitzen ein Asymmetriezentrum und liegen daher als Racemate vor. Diese Racemate können in üblicher Weise, z.B. durch Salzbildung mit optisch aktiven Säuren, in die entsprechenden Diastereomeren überführt werden, die dann in die optisch aktiven Endprodukte überführt werden können.

Die optisch aktiven Endprodukte erhält man auch direkt aus den optisch aktiven Aminoalkylverbindungen der Formel II.

Verfahren zur Herstellung der als Ausgangsstoffe verwendeten 4-Aminomethylpyrrolidinone der allgemeinen Formel II sind beschrieben im DPB ... (deutsche Patentanmeldung P 3 336 024.3). Man erhält diese Verbindungen beispielsweise, ausgehend von einem am Stick stoffatom entsprechend substituierten 4-Cyanomethylaminopyrrolidinon-(2), durch Hydrierung in üblicher Weise.

Nach den vorstehend genannten Verfahren können beispielsweise die folgenden Endprodukte erhalten werden:

4-(Methylamino-carbonylaminomethyl)-1-benzylpyrrolidin-2-on,
4-(Isopropylamino-carbonylaminomethyl)-1-benzylpyrrolidin-2-on,
4-(N,N-Dimethylaminoäthylamino-carbonylaminomethyl)-1-benzylpyrrolidin-2-on,
4-(Cyclohexylamino-carbonylaminomethyl)-1-benzylpyrrolidin-2-on,

4-(m-Chlorphenylamino-carbonylaminomethyl)-1-benzylpyrrolidin-2-on,
4-(N-Nortropanyl-carbonylaminomethyl)-1-benzylpyrrolidin-2-on,
4-(N-Morpholino-carbonylaminomethyl)-1-(p-methoxybenzyl)-pyrrolidin-2-on,
4-(N-Methylpiperazinyl-carbonylaminomethyl)-1-benzylpyrrolidin-2-on,
4-(N-Methylpiperazinyl-carbonylaminomethyl)-1-(p-methylbenzyl)-pyrrolidin-2-on,
4-(2,6-Dimethylmorpholino-carbonylaminomethyl)-1-(p-methylbenzyl)-pyrrolidin-2-on,
4-(N-Methylpiperazinyl-carbonylaminomethyl)-1-(o-chlorbenzyl)-pyrrolidin-2-on,
4-(N-Methylpiperazinyl-carbonylaminomethyl)-1-(p-fluorbenzyl)-pyrrolidin-2-on,
4-(N-Methylpiperazinyl-carbonyl-N′-methylaminomethyl)-1-benzylpyrrolidin-2-on,
4-(N-Methylpiperazinyl-carbonyl-N′-isopropylaminomethyl)-1-benzylpyrrolidin-2-on,
4-(Dimethylamino-carbonyl-N′-isopropyl-aminomethyl)-1-benzylpyrrolidin-2-on,
4-(Morpholino-carbonylaminomethyl)-1-benzylpyrrolidin-2-on,
4-(Piperazino-carbonylaminomethyl)-1-benzylpyrrolidin-2-on,
4-(N-(p-Chlorphenyl)-piperazino-carbonylaminomethyl)-1-benzylpyrrolidin-2-on,
4-(Piperidino-carbonylaminomethyl)-1-benzylpyrrolidin-2-on,
4-(β-Hydroxyäthylamino-carbonylaminomethyl)-1-benzylpyrrolidin-2-on,
4-(p-Chlorphenylamino-carbonylaminomethyl)-1-benzylpyrrolidin-2-on,
4-(N-Benzylpiperazinyl-carbonylaminomethyl)-1-benzylpyrrolidin-2-on.

Die Pyrrolidinon-Derivate wurden in Tierexperimenten bezüglich ihrer Wirksamkeit untersucht, Zustandsformen eingeschränkter cerebraler Leistungsfähigkeit aufzuheben beziehungsweise zu mindern.

Es wurde gefunden, dass sie mit hoher Affinität an muscarin-cholinerge Rezeptorstrukturen des Rattencortex binden. In Untersuchungen zur zentralen Aktivierung wurde im EEG von Katzen unter der Wirkung der neuen Verbindungen eine Desynchronisation (Weck- beziehungsweise Wachreaktion) gefunden. Hieraus könnte sich eine erwünschte zentralstimulierende Wirkung beim Menschen, insbesondere unter dem Aspekt des Krankheitsbildes verminderter cholinerger Überträgerfunktion (Alzheimersche Erkrankung) ergeben.

Die Verbindungen weisen in orientierenden Verträglichkeitsuntersuchungen an der Maus in Dosierungen bis zu 2 g/kg (einmalige orale Applikation) keine akute Toxizität (14 Tage Nachbeobachtung) auf. Sie zeigen tierexperimentell ausgezeichnete Wirkungen auf spontane kognitive Leistungen wie experimentell eingeschränkte Lern- und Gedächtnisvorgänge. In Versuchen mit Einschränkung des Kurzzeitgedächtnisses beziehungsweise Behinderung des Übergangs von Inhalten des Kurzzeit- in das Langzeitgedächtnis durch Gabe eines muscarinen cholinergen Antagonisten (Scopolamin 0,6 mg/kg i.p.; siehe auch Psychopharmacology 78, 104–111 (1982)), sind die Verbindungen in der Lage, dieser pharmakologisch induzierten cerebralen Insuffizienz entgegenzuwirken beziehungsweise sie aufzuheben.

Die Lernfähigkeit von Ratten in einer aktiven Vermeidedressur (J. pharmacol. Methods, 8, 255–263 (1983)) wird ebenso verbessert wie ihre Habituation beziehungsweise explorierende Orientierungsaktivität in einer neuen Umgebung.

Bei der Prüfung der Überlebensfähigkeit von Tieren in einer geschlossenen Kammer (Hypoxie-Toleranztest), welche mit einem Gasgemisch, bestehend aus 96,5% $N_2$ und 3,5% $O_2$ durchströmt wurde, wiesen die mit den neuen Substanzen vorbehandelten Tiere eine statistisch hochsignifikant grössere Überlebensfähigkeit auf als Kontrolltiere beziehungsweise mit Piracetam vorbehandelte Tiere. Darüber hinaus war die mit dieser Methode geprüfte hirnprotektive Wirkung der Substanzen bereits in einer Dosis von 100 mg/kg p.o. ausgeprägt.

Die erfindungsgemässen Pyrrolidinon-Derivate wurden in ihrer Wirksamkeit mit andersstrukturierten Pyrrolidinonen verglichen, welche im Rahmen der cerebralen Insuffizienz beziehungsweise des hirnorganischen Psychodroms, der posttraumatischen und alkoholischen Hirnschädigung usw., in der Humanmedizin bereits als Arzneimittel Anwendung finden (Pirazetam) beziehungsweise zurzeit klinisch erprobt werden (Anirazetam).

Die Verbindungen sind sowohl bezüglich der wirksamen Dosis als auch der im Tierexperiment erzielten Leistungsverbesserung den genannten Substanzen deutlich überlegen.

Die anmeldungsgemässen Verbindungen können allein oder in Kombination mit anderen erfindungsgemässen Wirkstoffen, gegebenenfalls auch in Kombination mit weiteren pharmakologisch aktiven Wirkstoffen, z.B. weiteren Cerebroaktivatoren zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln wie Maisstärke oder Alginsäure, Bindemitteln wie Stärke oder Gelatine, Schmiermitteln wie Magnesiumstearat oder Talk, und/oder Mitteln zur Erzielung des Depoteffektes wie Carboxypolymethylen, Carboxymethylcellulose, Celluloseacetatphthalat oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemässen Wirkstoffe beziehungsweise Wirkstoffkombinationen können zusätzlich noch ein Süssungsmittel wie Saccharin, Cyclamat, Glycerin oder Zucker sowie ein geschmacksverbesserndes Mittel, z.B. Aromastoffe wie Vanillin oder Orangenextrakt enthalten. Sie können ausserdem Suspendierhilfsstoffe oder Dickungsmittel, wie Natriumcarboxymethylcellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Äthylendiamintetraessigsäure, hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Die nachfolgenden Beispiele illustrieren die vorliegende Erfindung, ohne sie jedoch in ihrem Umfang zu beschränken:

Beispiel 1

4 - (N - Methylpiperazinyl - carbonylaminomethyl)-1-benzylpyrrolidin-2-on (nach Verfahren b))

36 g (0,18 Mol) 4-Aminomethyl-1-benzylpyrrolidin-2-on (s. DBP ..., deutsche Patentanmeldung P 3 336 024.3) werden in 500 ml trockenem Dioxan gelöst und während 30 Minuten unter Rühren mit 29 g (0,18 Mol) Chlorcarbonylmethylpiperazin versetzt. Man kocht 30 Minuten unter Rückfluss, wobei ein dunkles Öl ausfällt. Das Reaktionsgemisch wird eingedampft und der Rückstand unter Eiskühlung mit 2 n Natronlauge alkalisch gestellt. Man schüttelt die Titelverbindung mit Methylenchlorid aus und erhält nach Abdestillieren des Lösungsmittels ca. 50 g Rohprodukt. Nach Chromatographie über $SiO_2$ (Laufmittel Methylenchlorid/Methanol 98:2) und Umkristallisieren aus wenig Essigester werden 42 g farblose Kristalle vom Fp. 123–124°C erhalten (70% d.Th.).

33 g (0,1 Mol) der Base werden mit 280 ml Äthanol heiss gelöst und mit 11,5 g Fumarsäure versetzt. Das Fumarat kristallisiert aus, wird nach dem Abkühlen abgesaugt und mit kaltem Äthanol gewaschen. Ausbeute: 41 g (93% d.Th.), Fp. 182–184°C. Das Salz enthält ein Mol Fumarsäure.

Beispiel 2

(+) 4-(N-Methylpiperazinyl-carbonylaminomethyl)-1-benzylpyrrolidin-2-on

Nach dem in Beispiel 1 beschriebenen Verfahren erhält man die Titelverbindung als farbloses Öl, $\alpha_D^{20}$ + 1,8° (c = 10,0, Methanol) in einer Ausbeute von 40 g (= 68% d.Th.), ausgehend von 36 g (0,18 Mol) (−) 4-Aminomethyl-1-benzylpyrrolidin-2-on (hergestellt aus dem Racemat durch Antipodenspaltung mit Hilfe von Weinsäure ($\alpha_D^{20}$ − 2,1°, c = 10,0, Methanol) und gereinigt mittels Säulenchromatographie ($SiO_2$, Laufmittel: Methylenchlorid/Methanol 9:1).

Durch Umsetzung der optisch aktiven Base mit Fumarsäure erhält man 48 g (= 89% d.Th.) Fumarat vom Fp. 179–180°C. Das Salz enthält 1 Mol Fumarsäure.

Beispiel 3

(−) 4-(N-Methylpiperazinyl-carbonylaminomethyl)-1-benzylpyrrolidin-2-on

Ausgehend von (+) 4-Amino-1-benzylpyrrolidin-2-on ($\alpha_D^{20}$ + 2,07°; c = 10,0, Methanol) erhält man auf analoge Weise die Titelverbindung, $\alpha_D^{20}$ − 1,8° (c = 10,0, Methanol); Fumarat: Fp. 178–180°C.

Beispiel 4

4-(N-Methylpiperazinyl-carbonylaminomethyl)-1-benzylpyrrolidin-2-on (nach Verfahren c))

76 g (0,37 Mol) 4-Aminomethyl-1-benzylpyrrolidin-2-on werden in 1,2 l trockenem Dioxan gelöst und 52 ml Triäthylamin zugesetzt. Während 15–20 Minuten lässt man unter Eiskühlung 40 ml Chlorameisensäurephenylester zutropfen und dampft nach weiteren 30 Minuten im Vakuum ein. Der Rückstand wird in Methylenchlorid aufgenommen und die organische Lösung mehrmals mit Wasser gewaschen. Die getrocknete organische Phase wird über Kohle abgesaugt und eingedampft. Durch Zusatz von Äther kristallisieren 100 g 4-Phenoxycarbonylaminomethyl-1-benzylpyrrolidin-2-on (= 82% d.Th.) vom Fp. 89–90°C.

100 g dieser Verbindung werden mit 1,2 l Acetonitril und 62 g N-Methylpiperazin 2 Stunden unter Rückfluss gekocht. Man dampft das Lösungsmittel ab, nimmt in Methylenchlorid auf, wäscht mit Wasser, trocknet mit Magnesiumsulfat, dampft erneut ein und kristallisiert aus Essigester um. Ausbeute: 78 g (= 80% d.Th.), Fp. 124–126°C.

Beispiel 5

4 - (p - Chlorphenylamino - carbonylaminomethyl)-1-benzylpyrrolidin-2-on (nach Verfahren a))

52 g (0,25 Mol) 4-Aminomethyl-1-benzylpyrrolidin-2-on werden in 70 ml Dioxan mit 38 g (0,25 Mol) p-Chlorphenylisocyanat 2 Stunden bei Raumtemperatur gerührt und die Lösung anschliessend im Vakuum eingedampft. Der Rückstand kristallisiert aus Essigester. Ausbeute: 67 g (= 74% d.Th.), Fp. 139–140°C.

Beispiel 6

4 - (N - Methylpiperazinyl - carbonyl - N′ -methylaminomethyl)-1-benzylpyrrolidin-2-on (nach Verfahren d))

33 g (0,1 Mol) 4-(N-Methylpiperazinyl-carbonyl-aminomethyl)-1-benzylpyrrolidin-2-on, 500 ml Tetrahydrofuran und 4,5 g Natriumhydrid (Ölsuspension, 55%ig) werden 30 Minuten bei Raumtemperatur gerührt (Wasserstoffentwicklung). Man fügt 20 g Methyljodid zu (ca. 0,15 Mol) und erhitzt 3 Stunden unter Rückfluss. Danach wird das Reaktionsgemisch eingedampft, dem Rückstand Wasser zugefügt und mit Methylenchlorid ausgeschüttelt. Man erhält die Titelverbindung in einer Ausbeute von 16 g (= 50% d.Th.) vom Fp. 134–136°C.

Analog den in den Beispielen 1–6 beschriebenen Verfahren wurden die folgenden Verbindungen hergestellt:

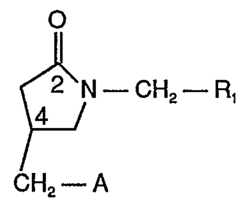

| $R_1$ | A | Fp. Base °C | Fumarat °C |
|---|---|---|---|
| 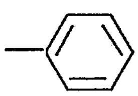 | $-\underset{H}{N}-CO-\underset{H}{N}-CH_3$ | 117—118 | |
| 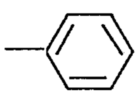 | 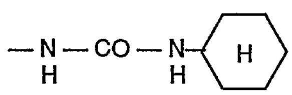 | 115—117 | |
| 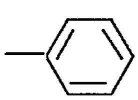 | 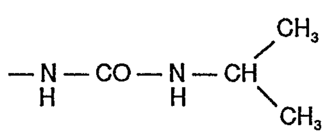 | 102—103 | |
| 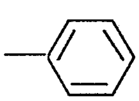 | 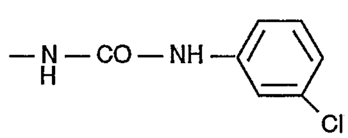 | 114—116 | |
| 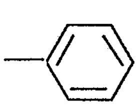 | 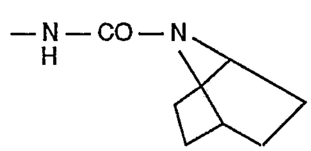 | 115—117 | |
| 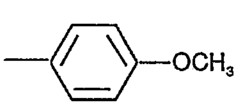 | 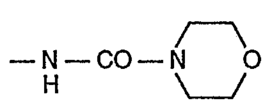 | 104—106 | |

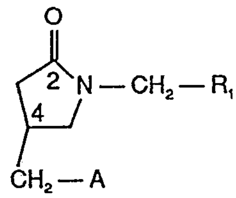

| $R_1$ | A | Fp. Base °C | Fumarat °C |
|---|---|---|---|
| 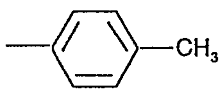 | 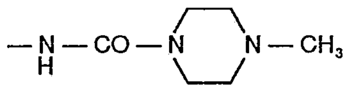 | 139—140 | |
| 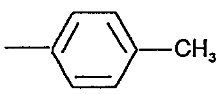 | 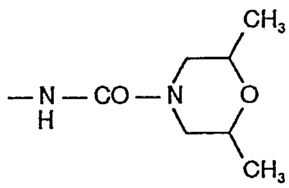 | 110—112 | |
| 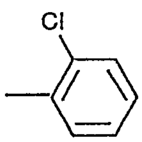 | 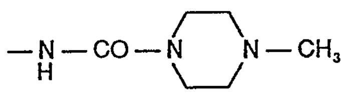 | | 178—180 |
| 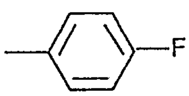 | 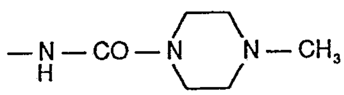 | | 153—154 |
| 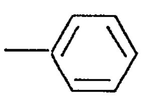 | 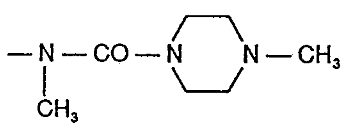 | 134—136 | |
| 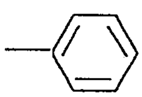 | 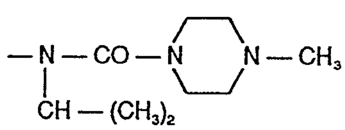 | 160—162 | |
| 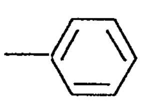 | 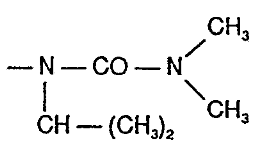 | 88—89 | |
| 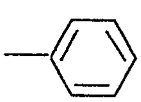 | 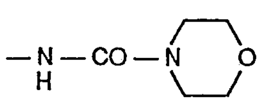 | 127—128 | |

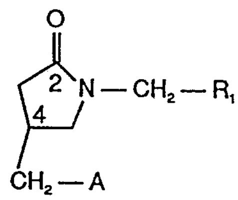

| $R_1$ | A | Fp. Base °C | Fumarat °C |
|---|---|---|---|
| 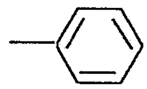 | 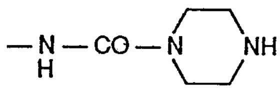 | Öl | |
| 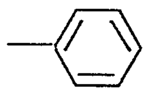 | 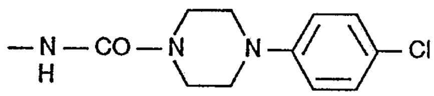 | 168—170 | |
| 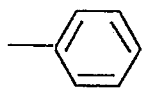 | 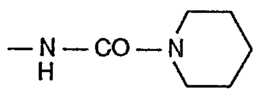 | 106—108 | |
| 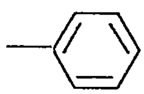 | $-\underset{H}{N}-CO-\underset{H}{N}-CH_2-CH_2-OH$ | Öl | |
| 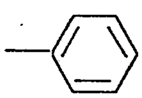 | 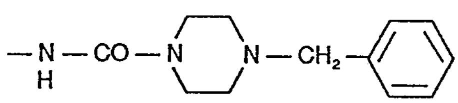 | 139—141 | |
| 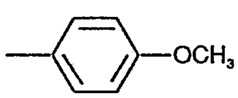 | 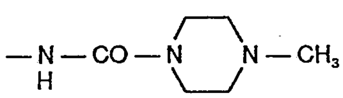 | 114—115 | |
| 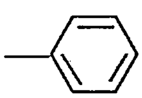 | 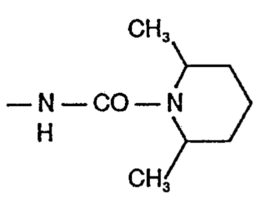 | 131—134 | |

Pharmazeutische Formulierungsbeispiele

A) Tabletten

| | pro Tablette |
|---|---|
| Wirkstoff | 100 mg |
| Milchzucker (pulverisiert) | 140 mg |
| Maisstärke | 240 mg |
| Polyvinylpyrrolidon | 15 mg |
| Magnesiumstearat | 5 mg |
| | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Grösse verpresst.

B) Tabletten

| | pro Tablette |
|---|---|
| Wirkstoff | 80 mg |
| Maisstärke | 190 mg |
| Milchzucker | 55 mg |
| Mikrokristalline Cellulose | 35 mg |
| Polyvinylpyrrolidon | 15 mg |
| Natrium-carboxymethylstärke | 23 mg |
| Magnesiumstearat | 2 mg |
| | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpresst das Gemisch zu Tabletten geeigneter Grösse.

C) Ampullen

| | | |
|---|---|---|
| 4-(N-Methylpiperazinyl-carbonylaminomethyl)-1-benzylpyrrolidin-2-on-Fumarat | | 50,0 mg |
| Natriumchlorid | | 10,0 mg |
| bidestilliertes Wasser | q.s. ad | 1,0 ml |

Herstellung:
Der Wirkstoff und das Natriumchlorid werden in bidestilliertem Wasser gelöst und die Lösung in Ampullen steril abgefüllt.

D) Tropfen

| | | |
|---|---|---|
| 4-(Isopropylamino-carbonylaminomethyl)-1-benzylpyrrolidin-2-on-Fumarat | | 50,0 mg |
| p-Hydroxybenzoesäuremethylester | | 0,1 g |
| p-Hydroxybenzoesäurepropylester | | 0,1 g |
| entmineralisiertes Wasser | q.s. ad | 100,0 ml |

Herstellung:
Der Wirkstoff und die Konservierungsmittel werden in demineralisiertem Wasser gelöst und die Lösung filtriert und in Flaschen zu je 100 ml abgefüllt.

**Patentansprüche**

1. Substituierte Pyrrolidinone der allgemeinen Formel

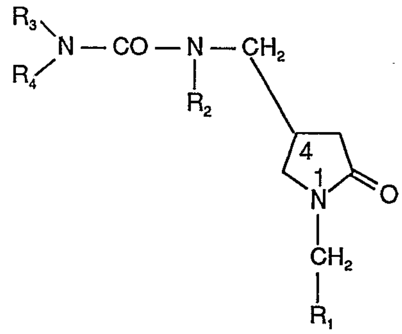

(I)

worin
$R_1$ einen Phenylrest, der ein- oder zweifach durch Methyl, Methoxy, Fluor, Chlor, Brom oder Trifluormethyl substituiert sein kann oder einen Pyridylrest;
$R_2$ Wasserstoff oder einen geradkettigen oder verzweigten Alkylrest mit 1-4 Kohlenstoffatomen;
$R_3$ einen geradkettigen oder verzweigten Alkylrest mit 1-3 Kohlenstoffatomen, einen Hydroxyalkylrest mit 2-3 Kohlenstoffatomen, einen Phenylrest, der ein- oder zweifach durch Chlor, Brom, Methyl oder Methoxy substituiert sein kann, einen Cyclohexylrest oder einen Dialkylaminoalkylrest, wobei jede Alkylgruppe 1-3 Kohlenstoffatome aufweisen kann;
$R_4$ Wasserstoff oder einen geradkettigen oder verzweigten Alkylrest mit 1-3 Kohlenstoffatomen, oder
$R_3$ und $R_4$ zusammen mit dem Stickstoffatom einen Piperidin-, Morpholin- oder Piperazinring, wobei die Ringe durch ein bis zwei Methylgruppen substituiert sein können und der Piperazinring am Stickstoffatom in 4-Stellung auch eine Phenyl-, Chlorphenyl- oder Benzylgruppe tragen kann, oder den Nortropanylrest bedeuten, sowie die physiologisch verträglichen Säureadditionssalze derjenigen Endprodukte, die eine basische Funktion im Molekül besitzen.

2. Substituierte Pyrrolidinone der allgemeinen Formel I gemäss Anspruch 1, worin $R_1$ den Phenylrest, $R_2$ Wasserstoff, $R_3$ einen Phenylrest, der gegebenenfalls in ortho- und/oder para-Stellung durch Chlor substituiert ist, $R_4$ Wasserstoff oder $R_3$ und $R_4$ zusammen mit dem Stickstoffatom einen gegebenenfalls ein- oder zweifach methylsubstituierten Morpholinyl-, Piperazinyl- oder Piperidinylrest bedeuten.

3. Substituierte Pyrrolidinone der allgemeinen Formel I gemäss Anspruch 1, worin $R_1$ Phenyl, $R_2$ Wasserstoff, $R_3$ Dialkylaminoalkyl mit jeweils 1 bis 3 Kohlenstoffatomen und $R_4$ Wasserstoff oder $R_3$ und $R_4$ zusammen mit dem Stickstoffatom einen Piperazinring bedeuten, der gegebenenfalls in 4-Stellung durch Methyl substituiert ist.

4. 4-(N-Methylpiperazinyl-carbonylaminome-

thyl)-1-benzylpyrrolidin-2-on und dessen Säureadditionssalze.

5. 4-(p-Chlorphenylamino-carbonylaminomethyl)-1-benzylpyrrolidin-2-on.

6. Verfahren zur Herstellung von Verbindungen der allgemeinen Formel I gemäss Anspruch 1, dadurch gekennzeichnet, dass man

a) zur Herstellung solcher Verbindungen der allgemeinen Formel I, in denen $R_4$ für Wasserstoff steht, ein Aminomethylpyrrolidin-2-on der allgemeinen Formel

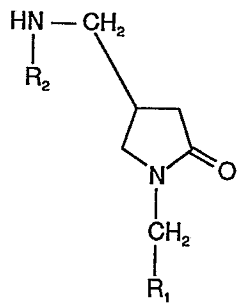

(II)

worin

$R_1$ und $R_2$ die in Anspruch 1 angegebene Bedeutung besitzen, mit einem Isocyanat der allgemeinen Formel

$O=C=N-R_3$ (III)

worin

$R_3$ die in Anspruch 1 angeführte Bedeutung mit Ausnahme der Bedeutung Hydroxyalkyl besitzt, umsetzt; oder dass man

b) eine Verbindung der allgemeinen Formel II mit einem Chlorcarbonylamid der allgemeinen Formel

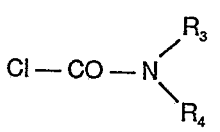

(IV)

worin

$R_3$ die in Anspruch 1 angegebene Bedeutung mit Ausnahme von Hydroxyalkyl und $R_4$ die in Anspruch 1 angegebene Bedeutung mit Ausnahme von Wasserstoff besitzen, umsetzt; oder dass man

c) eine Verbindung der allgemeinen Formel II mit einem Chlorkohlensäureester der allgemeinen Formel

$Cl-CO-O-Y$ (V)

worin

Y einen Alkylrest mit 1-4 Kohlenstoffatomen den Benzyl-, Phenyl- oder p-Nitrophenylrest bedeutet, zu einem Carbamat der allgemeinen Formel

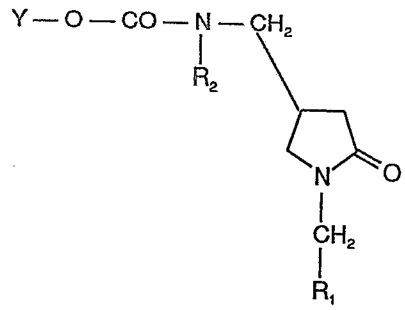

(VI)

worin

$R_1$, $R_2$ und Y die oben angeführte Bedeutung besitzen, umsetzt und dieses Carbamat mit einem Amin der allgemeinen Formel

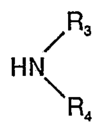

(VII)

worin

$R_3$ und $R_4$ die in Anspruch 1 angegebene Bedeutung besitzen, reagieren lässt; oder dass man

d) ein Endprodukt der allgemeinen Formel I, in dem $R_2$ und/oder $R_4$ Wasserstoff bedeuten, in üblicher Weise alkyliert, und dass man solche Endprodukte der allgemeinen Formel I, die eine basische Funktion im Molekül besitzen, in ihre physiologisch verträglichen Säureadditionssalze überführt.

7. Racemate und optisch aktive Formen von Verbindungen der allgemeinen Formel I gemäss Anspruch 1.

8. Pharmazeutische Zubereitungen, enthaltend als Wirkstoff eine oder mehrere Verbindungen der allgemeinen Formel I gemäss Anspruch 1 oder deren physiologisch verträgliche Säureadditionssalze in Kombination mit üblichen Hilfs- und/oder Trägerstoffen.

9. Verfahren zur Herstellung von pharmazeutischen Präparaten gemäss Anspruch 8, dadurch gekennzeichnet, dass man Verbindungen der allgemeinen Formel I mit üblichen galenischen Hilfs- und/oder Trägerstoffen zu üblichen pharmazeutischen Anwendungsformen verarbeitet.

**Claims**

1. Substituted pyrrolidinones of general formula

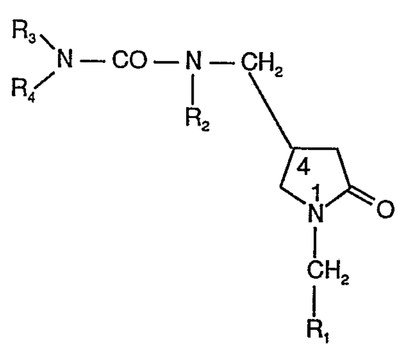

(I)

wherein
$R_1$ represents a phenyl group which may be mono- or disubstituted by methyl, methoxy, fluorine, chlorine, bromine or trifluoromethyl, or a pyridyl group;
$R_2$ represents hydrogen or a straight-chained or branched alkyl group with 1-4 carbon atoms;
$R_3$ represents a straight-chained or branched alkyl group with 1-3 carbon atoms, a hydroxyalkyl group with 2-3 carbon atoms, a phenyl group, which may be mono- or disubstituted by chlorine, bromine, methyl or methoxy, a cyclohexyl group or a dialkylaminoalkyl group, whilst each alkyl group may contain from 1-3 carbon atoms;
$R_4$ represents hydrogen or a straight-chained or branched alkyl group with 1-3 carbon atoms, or $R_3$ and $R_4$ together with the nitrogen atom may represent a piperidine, morpholine or piperazine ring, whilst the rings may be substituted by one to two methyl groups and the piperazine ring may also carry a phenyl, chlorophenyl or benzyl group at the nitrogen atom in the 4 position or $R_3$ and $R_4$ may represent a nortropanyl group, and the physiologically acceptable acid addition salts of those end products which have a basic function in the molecule.

2. Substituted pyrrolidinones of general formula I as claimed in claim 1, wherein $R_1$ represents a phenyl group, $R_2$ represents hydrogen, $R_3$ represents a phenyl group which is optionally substituted by chlorine in the ortho and/or para position, $R_4$ represents hydrogen or $R_3$ and $R_4$ together with the nitrogen atom represent an optionally mono- or di-methyl substituted morpholinyl, piperazinyl or piperidinyl group.

3. Substituted pyrrolidinones of general formula I as claimed in claim 1, wherein $R_1$ represents phenyl, $R_2$ represents hydrogen, $R_3$ represents dialkylaminoalkyl each having 1 to 3 carbon atoms and $R_4$ represents hydrogen or $R_3$ and $R_4$ together with the nitrogen atom represent a piperazine ring which is optionally methyl-substituted in the 4 position.

4. 4-(N-methylpiperazinyl-carbonylaminomethyl)-1-benzyl-pyrrolidin-2-one and the acid addition salts thereof.

5. 4-(p-chlorophenylamino-carbonylaminomethyl)-1-benzyl-pyrrolidin-2-one.

6. Process for preparing compounds of general formula I as claimed in claim 1, characterised in that

a) in order to prepare compounds of general formula I wherein $R_4$ represents hydrogen, an aminomethyl-pyrrolidin-2-one of general formula

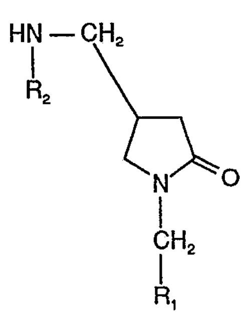

(II)

wherein
$R_1$ and $R_2$ have the meanings given in claim 1, is reacted with an isocyanate of general formula

$$O=C=N-R_3 \qquad (III)$$

wherein
$R_3$ has the meaning given in claim 1 with the exception of the definition hydroxyalkyl; or

b) a compound of general formula II is reacted with a chlorocarbonylamide of general formula

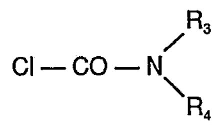

(IV)

wherein
$R_3$ has the meaning given in claim 1 with the exception of hydroxyalkyl and $R_4$ has the meaning given in claim 1 with the exception of hydrogen; or

c) a compound of general formula II is reacted with a chlorocarbonate of general formula

$$Cl\text{-}CO\text{-}O\text{-}Y \qquad (V)$$

wherein
Y represents an alkyl group with 1-4 carbon atoms, a benzyl, phenyl or p-nitrophenyl group, to form a carbamate of general formula

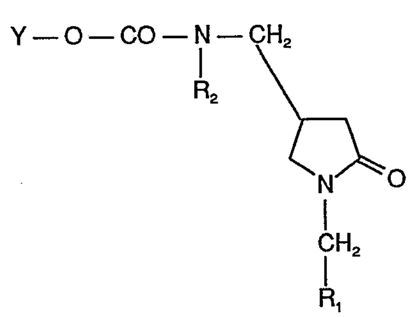

(VI)

wherein
$R_1$, $R_2$ and Y are defined as herein before, and this carbamate is reacted with an amine of general formula

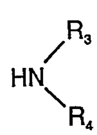

(VII)

wherein
$R_3$ and $R_4$ have the meanigns given in claim 1; or

d) an end product of general formula I wherein $R_2$ and/or $R_4$ represent hydrogen is alkylated in the usual way, and end products of general formula I which have a basic function in the molecule are converted into the physiologically acceptable acid addition salts thereof.

7. Racemates and optically active forms of compounds of general formula I as claimed in claim 1.

8. Pharmaceutical preparations containing as active substance one or more compounds of general formula I as claimed in claim 1 or the physiologically acceptable acid addition salts thereof in conjunction with conventional excipients and/or carriers.

9. Process for preparing pharamceutical preparations as claimed in claim 8, characterised in that compounds of general formula I are processed with conventional galenic excipients and/or carriers to produce conventional pharmaceutical forms for administration.

**Revendications**

1. Pyrrolidones substituées de formule générale

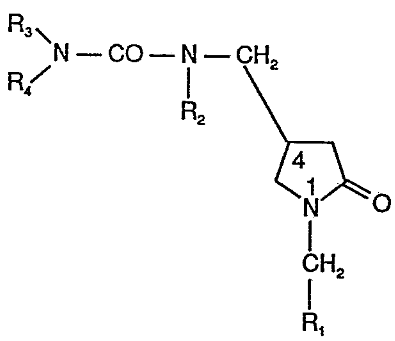

(I)

dans laquelle

$R_1$ représente un radical phényle, qui peut être substitué une ou deux fois par méthyle, méthoxy, fluor, chlore, brome ou trifluorométhyle ou représente un radical pyridyle;

$R_2$ représente l'hydrogène ou un radical alcoyle, rectiligne ou ramifié, avec 1-4 atomes de carbone;

$R_3$ représente un radical alcoyle, rectiligne ou ramifié, avec 1-3 atomes de carbone, un radical hydroxyalcoyle avec 2-3 atomes de carbone, un radical phényle, qui peut être substitué une ou deux fois par chlore, brome, méthyle ou méthoxy, ou représente un radical cyclohexyle ou un radical dialcoylamino-alcoyle, chaque groupe alcoyle pouvant présenter 1-3 atomes de carbone;

$R_4$ représente l'hydrogène ou un radical alcoyle, rectiligne ou ramifié, avec 1-3 atomes de carbone; ou

$R_3$ et $R_4$, ensemble avec l'atome d'azote, représentent un cycle pipéridine, morpholine ou pipérazine, les cycles pouvant être substitués par un à deux groupes méthyle et le cycle pipérazine pouvant porter, sur l'atome d'azote en position 4, également un groupe phényle, chlorophényle ou benzyle, ou représentent le radical nortropanyle, ainsi que les sels d'addition d'acides physiologiquement supportables des produits finals qui possèdent une fonction basique dans la molécule.

2. Pyrrolidones substituées de formule générale I selon la revendication 1, dans laquelle $R_1$ représente le radical phényle, $R_2$ représente l'hydrogène, $R_3$ représente un radical phényle qui est éventuellement substitué en position ortho et/ou para par le chlore, $R_4$ représente l'hydrogène ou $R_3$ et $R_4$, ensemble avec l'atome d'azote, représentent un radical morpholinyle, pipérazinyle ou pipéridinyle, éventuellement une ou deux fois méthylsubstitué.

3. Pyrrolidones substituées de formule générale I selon la revendication 1, dans laquelle $R_1$ représente phényle, $R_2$ représente l'hydrogène, $R_3$ représente dialcoylamino-alcoyle avec à chaque fois 1-3 atomes de carbone et $R_4$ représente l'hydrogène ou $R_3$ et $R_4$, ensemble avec l'atome d'azote, représentent un cycle pipérazine qui est éventuellement substitué en position 4 par méthyle.

4. La 4-(N-méthylpipérazinyl-carbonylaminométhyl)-1-benzylpyrrolidine-2-one et ses sels d'addition d'acides.

5. La 4-(p-chlorophénylamino-carbonylaminométhyl)-1-benzylpyrrolidine-2-one.

6. Procédé pour la préparation des composés de formule générale I selon la revendication 1, caractérisé en ce que

a) pour la préparation de composés de formule générale I dans lesquels $R_4$ remplace l'hydrogène, on fait réagir une aminométhylpyrrolidine-2-one de formule générale

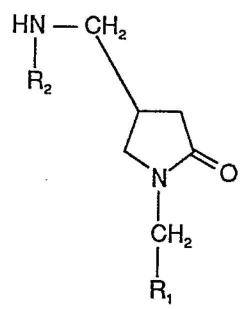

(II)

dans laquelle

$R_1$ et $R_2$ possèdent la signification donnée à la revendication 1, avec un isocyanate de formule générale

$$O{=}C{=}N{-}R_3 \quad (III)$$

dans laquelle

$R_3$ possède la signification indiquée à la revendication 1, à l'exception de la signification hydroxyalcoyle; ou en ce que

b) on fait réagir un composé de formule générale II avec un chlorocarbonylamide de formule générale

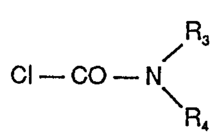

(IV)

dans laquelle
$R_3$ possède la signification donnée à la revendication 1, à l'exception d'hydroxyalcoyle et $R_4$ possède la signification donnée à la revendication 1, à l'exception de l'hydrogène; ou en ce que

c) on fait réagir un composé de formule générale II avec un ester d'acide chlorocarbonique de formule générale

Cl-CO-O-Y (V)

dans laquelle
Y représente un groupe alcoyle avec 1-4 atomes de carbone, le radical benzyle, phényle ou p-nitrophényle, pour donner un carbamate de formule générale

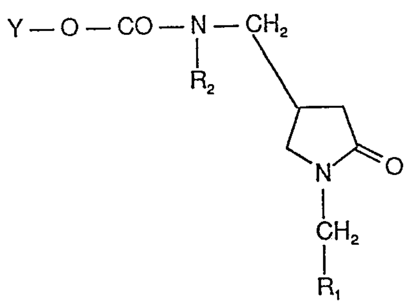

(VI)

dans laquelle
$R_1$, $R_2$ et Y possèdent la signification indiquée plus haut, et on laisse réagir ce carbamate avec une amine de formule générale

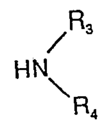

dans laquelle
$R_3$ et $R_4$ possèdent la signification donnée à la revendication 1; ou en ce que

d) on alcoyle de manière usuelle un produit final de formule générale I dans lequel $R_2$ et/ou $R_4$ représentent l'hydrogène, et en ce qu'on transforme les produits finals de formule générale I qui possèdent une fonction basique dans la molécule, en leurs sels d'addition d'acides physiologiquement supportables.

7. Racémates et formes optiquement actives des composés de formule générale I selon la revendication 1.

8. Préparations pharmaceutiques contenant, en tant que substance active, un ou plusieurs composés de formule générale I selon la revendication 1 ou leurs sels d'addition d'acides physiologiquement supportables, en combinaison avec des adjuvants et/ou excipients usuels.

9. Procédé pour la fabrication des préparations pharmaceutiques selon la revendication 8, caractérisé en ce qu'on transforme des composés de formule générale I, au moyen d'adjuvants et/ou excipients galéniques usuels, en formes d'utilisation pharmaceutiques usuelles.